# EUROPEAN PATENT SPECIFICATION

(11) **EP 2 387 985 B1**
(45) Date of publication and mention of the grant of the patent: **02.04.2014**
(21) Application number: 10731271.2
(22) Date of filing: 14.01.2010
(51) Int. Cl.: A61K 8/06, A61K 8/34, A61K 8/86, A61K 9/107, A61K 47/10, A61K 47/34, A61Q 19/00, B01J 13/00

(54) **PROCESS FOR PRODUCING O/W MICROEMULSION PREPARATION FOR EXTERNAL APPLICATION**
VERFAHREN ZUR HERSTELLUNG EINES Ö/W-MIKROEMULSIONSPRÄPARATS ZUR EXTERNEN ANWENDUNG
PROCÉDÉ DE PRODUCTION D'UNE PRÉPARATION DE MICROÉMULSION D'HUILE DANS L'EAU POUR APPLICATION EXTERNE

(30) Priority: 14.01.2009 JP 2009005531
(43) Date of publication of application: 23.11.2011
(73) Proprietor: Shiseido Company Ltd., Chuo-ku Tokyo 104-0061 (JP)
(72) Inventor: MIYAHARA, Reiji, Yokohama-shi Kanagawa 236-8643 (JP); ARAKI, Hidefumi, Yokohama-shi Kanagawa 224-8558 (JP)
(74) Representative: Henkel, Breuer & Partner
(86) International application number: PCT/JP2010/050341
(87) International publication number: WO 2010/082602

(56) References cited:
- EP-A1- 1 702 607
- JP-A- 1 155 941
- JP-A- 5 505 559
- JP-A- 2005 193 134
- JP-A- 2007 254 405
- DATABASE WPI Week 200373 Thomson Scientific, London, GB; AN 2003-770847 XP002677679, & JP 2003 012492 A (NOEVIR KK) 15 January 2003 (2003-01-15)
- DATABASE WPI Week 200821 Thomson Scientific, London, GB; AN 2008-C88727 XP002677680, & JP 2007 254405 A (SHISEIDO CO LTD) 4 October 2007 (2007-10-04)
- DATABASE WPI Week 200554 Thomson Scientific, London, GB; AN 2005-525888 XP002677681, & JP 2005 193134 A (SHISEIDO CO LTD) 21 July 2005 (2005-07-21)
- DATABASE WPI Week 198930 Thomson Scientific, London, GB; AN 1989-217410 XP002677682, & JP 1 155941 A (LION CORP) 19 June 1989 (1989-06-19)

## Description

### RELATED APPLICATIONS

This application claims the priority of Japanese Patent Application No. 2009-5531 filed on January 14, 2009.

### FIELD OF THE INVENTION

The present invention relates to a production method of a fine O/W emulsion external preparation. In particular, it relates to a simple and economical production method and to improvement in stability of a fine O/W emulsion external preparation.

### BACKGROUND OF THE INVENTION

In the past, in order to maintain the emulsion stability of skin external preparations such as cosmetics, quasi-drugs, and pharmaceuticals, O/W emulsion compositions with the use of an α-gel, which is formed from a higher aliphatic alcohol and a polyoxyethylene-type nonionic surfactant, have been used and known especially for use as external preparations. As the preparation method of such an O/W emulsion external preparation, the following method has been used: a moisturizer and a hydrophilic polyoxyethylene-type nonionic surfactant are dissolved in water and heated to about 70 °C to prepare a water phase; an oil component and a higher alcohol, as essential components, were homogeneously mixed at about 70 °C to prepare an oil phase; the oil phase is emulsified by stirring with a homogenizer into the water phase; and the emulsion is rapidly cooled with a cooling machine such as an Onlator to about 35 °C (for example, refer to Non-patent Literature 1).

However, the thus far investigated method comprising emulsification at 70 °C followed by cooling has issues in that not only the method is energetically wasteful but also a large amount of water is used during washing after the use of a cooling machine such as an Onlator. In addition, it is difficult to prepare an emulsion having the particle size of 1 µm or less. In recent years, a low-energy emulsification technology has been sought-after because of increased environmental awareness.

Accordingly, the development of an O/W emulsion external preparation that can be economically and easily produced without using a conventional cooling apparatus such as an Onlator, and has long-term stability and smaller particle sizes, has been awaited.

### RELATED ART DOCUMENT

### NON-PATENT LITERATURE

Non-patent Literature 1: "Chemistry and Application of Surface Activity" (in Japanese) written by Manabu Senoo, Dainippon Tosho Co., Ltd., 1995; p 160.

### DISCLOSURE OF THE INVENTION

### PROBLEM TO BE SOLVED BY THE INVENTION

The present invention was made in view of the above-described situation of the conventional art. The problems to be solved are to provide a simple and economical production method of a fine O/W emulsion external preparation and to provide a fine O/W emulsion external preparation excellent in stability.

### MEANS TO SOLVE THE PROBLEM

In view of the above-described problems, the present inventors have diligently repeated studies. As a result, the present inventors have found that a fine O/W emulsion external preparation having the emulsion particle size of 50 to 500 nm with excellent stability can be produced without using a cooling apparatus such as an Onlator. This was achieved by mixing with stirring a specific composition at 70 to 80 °C to prepare a W/O emulsion and then by adding water or an aqueous formulation at 10 to 35 °C to the W/O emulsion while mixing with stirring to invert the W/O emulsion into a fine O/W emulsion. Furthermore, the present inventors have found that the obtained fine O/W emulsion can provide a light and fresh feeling during application but a moist and rich texture after application, though these properties could not be achieved by the conventional preparation method with the use of the Onlator, thus leading to completion of the present invention.

That is, the production method of a fine O/W emulsion external preparation of the present invention is a production method of a fine O/W emulsion external preparation having the emulsion particle size of 50 to 500 nm comprising the steps of:
mixing with stirring, at 70 to 80 °C,
   (A) a hydrophilic nonionic surfactant,
   (B) a linear higher alcohol having 16 or more carbon atoms,
   (C) an oil component,
   (D) an aqueous solvent which can be soluble in water, wherein the critical micelle concentration (c.m.c.) of the hydrophilic nonionic surfactant in the aqueous solvent is higher than that in water, and
   (E) water in the amount of 5 to 25 mass % of the total amount of (A) to (E), to prepare a W/O emulsion; and
adding, while mixing with stirring, (F) water or an aqueous formulation at 10 to 35 °C into the W/O emulsion to invert the W/O emulsion to a fine O/W emulsion.

In the production method of a fine O/W emulsion external preparation, it is preferable that the HLB of (A) the hydrophilic nonionic surfactant is 8 or higher.

The mass ratio of (A) the hydrophilic nonionic surfactant and (B) the linear higher alcohol having 16 or more carbon atoms is 3:7 to 7:3.

The oil component is 1 to 40 mass % in the W/O emulsion.
The aqueous solvent is 5 mass % or higher in the W/O emulsion.

In any of the production methods of a fine O/W emulsion external preparation, it is preferable that (D) the aqueous solvent has three or less hydroxyl groups in the molecule.

In any of the production methods of a fine O/W emulsion external preparation, it is preferable that (D) the aqueous solvent is one or more selected from the group consisting of polypropylene glycol polyethylene glycol copolymer or its dimethyl ether, polyethylene glycol or its alkyl ethers, dipropylene glycol, isoprene glycol, and propylene glycol.

### EFFECT OF THE INVENTION

According to the production method of a fine O/W emulsion external preparation of the present invention, the emulsion can be easily produced with low-energy without using a cooling machine such as an Onlator; thus it is very economical. In addition, the fine O/W emulsion external preparation, produced by the production method of the present invention, is essentially obtained by emulsification only with a nonionic surfactant whose irritation to the human body is relatively small; thus, it is very safe to the human body. Furthermore, the particle size of the fine O/W emulsion external preparation, produced by the production method of the present invention, is very small ranging 50 to 500 nm; nevertheless, the stability is excellent. In addition, when compared with the O/W emulsion produced with the use of a cooling machine such as an Onlator, the present external preparation, regardless of the identical formulation, provides light and fresh feeling but also provides a rich feeling.

### BRIEF DESCRIPTION OF THE DRAWINGS

Fig 1 shows a conceptual diagram of the production method of a fine O/W emulsion external preparation of the present invention.
Fig. 2 shows 400 times magnification micrographs of emulsion particles produced in Production Example 1-1 in accordance with the present invention and emulsion particles produced in Comparative Example 1-1.
Fig. 3 shows 400 times magnification micrographs of emulsion particles produced in Production Example 1-2 in accordance with the present invention and emulsion particles produced in Comparative Example 1-2.
Fig. 4 shows 400 times magnification micrographs of emulsion particles produced in Production Examples 2-2 to 2-7 in accordance with the present invention.
Fig. 5 shows 400 times magnification micrographs of emulsion particles produced in Production Examples 3-4 to 3-8 in accordance with the present invention.
Fig. 6 shows 400 times magnification micrographs of emulsion particles produced in Production Examples 5-1 to 5-5 in accordance with the present invention.

### MODE FOR CARRYING OUT THE INVENTION

Hereinafter, the constitution of the present invention will be described in detail.

The fine O/W emulsion external preparation of the present invention is prepared by the emulsification method comprising the steps according to claim 1.

In the present invention, the "fine O/W emulsion" is an emulsion system consisting of two liquid phases (in which the oil phase is emulsified in the water phase) for a water-surfactant-oil system. It is a thermodynamically unstable composition in which the emulsion particles are very fine to the extent that the emulsion is translucent or faint blue-white color.

(A) a hydrophilic nonionic surfactant used in the present invention is not limited in particular; however, it is the hydrophilic nonionic surfactant which can be soluble in the aqueous solvent by micellar dissolution. In particular, the hydrophilic nonionic surfactant having the HLB of 8 or higher is preferable. If the HLB of (A) the hydrophilic nonionic surfactant is smaller than 8, the preferable formation of the fine O/W emulsion may not be achieved.

Examples of (A) a hydrophilic nonionic surfactant used in the present invention include polyoxyethylene glycerol fatty acid esters, polyoxyethylene methylpolysiloxane copolymer, polyoxyethylene sorbitan fatty acid esters, polyoxyethylene alkyl ethers, maltitol hydroxy aliphatic alkyl ethers, alkylated polysaccharides, alkyl glucosides, sucrose fatty acid esters, and polyoxyethylene hydrogenated castor oil. Preferable examples thereof include polyoxyethylene-added nonionic surfactants. Two or more hydrophilic nonionic surfactants can be used.

As (B) a linear higher alcohol having 16 or more carbon atoms used in the present invention, those normally usable in the external preparations such as cosmetics and pharmaceuticals may be appropriately used, and two or more can be used in combination. Preferable examples thereof include saturated or unsaturated linear higher alcohols having 16 to 24 carbon atoms, such as cetyl alcohol, stearyl alcohol, behenyl alcohol, and batyl alcohol.

The mass ratio of (A) the hydrophilic nonionic surfactant and (B) the linear higher alcohol having 16 or more carbon atoms used in the present invention is 3:7 to 7:3. If the ratio deviates from this range, a stable fine O/W emulsion may not be formed.

The sum of (A) the hydrophilic nonionic surfactant and (B) the linear higher alcohol having 16 or more carbon atoms in the W/O emulsion is 0.5 to 10 parts by mass with respect to the 10 parts by mass of (C) the oil component. If the amount is less than 0.5 parts by mass, the amount of surfactant and higher alcohol is too small, whereby a fine O/W emulsion with high stability may not be obtained. If the amount exceeds 10 parts by mass, the amount of surfactant and higher alcohol is too much, whereby the usability tends to be undesirable.

(C) the oil component used in the present invention is not limited in particular and the oil components normally usable in the external preparations such as cosmetics and pharmaceuticals can be appropriately used. Examples thereof include silicone oils, hydrocarbon oils, ester oils, liquid fats, solid fats, waxes, higher fatty acids and higher alcohols (excluding the above-described (B)). Two or more of these may also be used in combination.

Examples of silicone oils include: linear polysiloxanes such as dimethylpolysiloxane, methylphenylpolysiloxane, and diphenylpolysiloxane; cyclic polysiloxanes such as octamethylcyclotetrasiloxane, decamethylcyclopentasiloxane, and dodecamethylcyclohexasiloxane; silicone resins having three-dimensional network structure; silicone rubber; and various modified polysiloxanes such as amino-modified polysiloxanes, polyether-modified polysiloxanes, alkyl-modified polysiloxanes, and fluorine-modified polysiloxanes.

Examples of hydrocarbon oils include liquid paraffin, ozokerite, squalane, pristane, paraffin, ceresin, squalene, petrolatum, and microcrystalline wax.

Examples of synthetic ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, acetylated lanolin, isocetyl stearate, isocetyl isostearate, cholesteryl 12-hydroxystearate, ethylene glycol di-2-ethylhexanoate, dipentaerythritol fatty acid esters, N-alkyl glycol monoisostearate, neopentyl glycol dicaprate, diisostearyl malate, glyceryl di-2-heptylundecanoate, trimethylolpropane tri-2-ethylhexanoate, trimethylolpropane triisostearate, pentaerythrityl tetra-2-ethylhexanoate, glyceryl tri-2-ethylhexanoate, glyceryl trioctanoate, glyceryl triisopalmitate, trimethylolpropane triisostearate, cetyl 2-ethylhexanoate, 2-ethylhexyl palmitate, glyceryl trimyristate, glyceryl tri-2-heptylundecanoate, castor oil fatty acid methyl ester, oleyl oleate, acetoglyceride, 2-heptylundecyl palmitate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, di-2-hexyldecyl adipate, diisopropyl sebacate, di-2-ethylhexyl succinate, and triethyl citrate.

Examples of liquid fats include avocado oil, camellia oil, turtle oil, macadamia nut oil, corn oil, mink oil, olive oil, rapeseed oil, egg oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, china paulownia oil, Japanese paulownia oil, jojoba oil, germ oil, and triglycerin.

Examples of solid fats include cacao butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oil, neat's-foot oil, Japan wax, and hydrogenated castor oil.

Examples of waxes include beeswax, candelilla wax, cotton wax, carnauba wax, bayberry wax, ibota wax, spermaceti wax, montan wax, rice bran wax, lanolin, kapok wax, acetylated lanolin, lanolin oil, sugar cane wax, lanolin fatty acid isopropyl ester, hexyl laurate, hydrogenated lanolin, jojoba wax, lanolin wax, shellac wax, POE lanolin alcohol ether, POE lanolin alcohol acetate, POE cholesterol ether, lanolin fatty acid polyethylene glycol, and POE hydrogenated lanolin alcohol ether.

Examples of higher fatty acids include lauric acid, myristic acid, palmitic acid, stearic acid, behenic acid, oleic acid, undecylenic acid, tall oil fatty acid, isostearic acid, linoleic acid, linolenic acid, eicosapentaenoic acid (EPA), and docosahexaenoic acid (DHA).

Examples of higher alcohols include: linear alcohols such as lauryl alcohol, and myristyl alcohol; and branched-chain alcohols such as 2-decyltetradecynol, lanolin alcohol, cholesterol, phytosterol, hexyldodecanol, isostearyl alcohol, and octyldodecanol.

The concentration of (C) the oil component used in the present invention is not limited in particular; however, it is preferable that the concentration is 1 to 40 mass % with respect to the total amount of the W/O emulsion. If the concentration exceeds 40 mass %, it is difficult to obtain a W/O emulsion with high stability.

(D) the aqueous solvent used in the present invention means a substance that is liquid at room temperature and miscible with water. In addition, it is necessary that, the aqueous solvent is immiscible with (C) the oil component and the critical micelle concentration (c.m.c.) of (A) the hydrophilic nonionic surfactant in the aqueous solvent is higher than that in water. Such (D) an aqueous solvent can lower the melting point of α-gel that is formed of (A) a hydrophilic nonionic surfactant and (B) a higher alcohol. Thus, when the W/O emulsion is prepared at 70 to 80 °C together with (D) an aqueous solvent and then (F) water or an aqueous formulation at 10 to 35 °C was added and mixed to the W/O emulsion, a fine stable O/W emulsion can easily be obtained without gelation. When such (D) an aqueous solvent is not used, when (D) an aqueous solvent is miscible with (C) an oil component, or when the critical micelle concentration (c.m.c.) of (A) a hydrophilic nonionic surfactant in the aqueous solvent is lower than that in water, a stable fine O/W emulsion cannot be obtained even if all other conditions are met.

(D) the aqueous solvent is not limited in particular if it is as those described above, and it is appropriately selected for use from publicly known aqueous solvents in accordance with the kinds of (C) an oil component and (A) a hydrophilic nonionic surfactant.

For an aqueous solvent, whether the critical micelle concentration (c.m.c.) of (A) a hydrophilic nonionic surfactant in the aqueous solvent is higher than that in water is determined by comparing the two critical micelle concentrations (c.m.c.) after measuring the critical micelle concentration (c.m.c.) of (A) the hydrophilic nonionic surfactant under the two conditions, namely in the aqueous solvent and in water. In many cases of aqueous solvents, however, it is very difficult to measure the critical micelle concentration (c.m.c.) of a hydrophilic nonionic surfactant in an aqueous solvent alone. In such cases, an aqueous solvent-water solution is prepared by adding a suitable amount of an aqueous solvent into water, the critical micelle concentration (c.m.c.) of (A) a hydrophilic nonionic surfactant is measured under the two conditions, namely in the aqueous solvent-water solution and in water (alone), and then both results may be compared. If the value of the critical micelle concentration (c.m.c.) of (A) a hydrophilic nonionic surfactant in the aqueous solvent-water solution is higher than the value of the critical micelle concentration (c.m.c.) in water, it can be determined that the aqueous solvent provides a higher critical micelle concentration (c.m.c.) of (A) the hydrophilic nonionic surfactant therein than that in water.

More specifically, an aqueous solvent is dissolved in water to be 10% to prepare an aqueous solvent-water solution, and when the value of the critical micelle concentration (c.m.c.) of (A) a hydrophilic nonionic surfactant in the 10% aqueous solvent-water solution is higher than the value of the critical micelle concentration (c.m.c.) in water by 30% or more, it can be determined that the aqueous solvent provides a higher critical micelle concentration (c.m.c.) of (A) the hydrophilic nonionic surfactant therein than that in water.

For example, when (A) the hydrophilic nonionic surfactant is POE(5) dodecyl ether, the critical micelle concentration (c.m.c.) in 10% POE(17) POP(4) dimethyl ether-water solution is 1.2x 10⁻⁴ mol/L at 25 °C, which is 84% higher than the critical micelle concentration in water, 6.5x10⁻⁵ mol/L. Thus, it can be said that POE(17) POP(4) dimethyl ether is an aqueous solvent that provides a higher critical micelle concentration (c.m.c.) of (A) the hydrophilic nonionic surfactant therein than that in water.

Furthermore, it is preferable that (D) an aqueous solvent used in the present invention provides a higher critical micelle concentration (c.m.c.) of (A) a hydrophilic nonionic surfactant therein. Specifically, it is preferable that the value of the critical micelle concentration (c.m.c.) of (A) a hydrophilic nonionic surfactant in 10% aqueous solvent-water solution is higher than the value of the critical micelle concentration (c.m.c.) in water by 50% or more.

Specific examples of (D) an aqueous solvent used in the present invention include aqueous solvents having three or less hydroxyl groups in the molecule. More specific examples include poly-(C2-C4)alkylene glycol polymer or its (C1-C5)alkyl ethers such as polypropylene glycol (1 to 20 mol) polyethylene glycol (5 to 30 mol) copolymer or its (C1 to C5)alkyl ethers and polyethylene glycol (1 to 50 mol) or its (C1-C5)alkyl ethers, polyoxy-(C2-C4)alkylene (2 to 30 mol) di-(C1-C5)alkylcarboxylates, 1,3-butylene glycol, dipropylene glycol, isoprene glycol, 1,2-pentane glycol, 1,2-hexane glycol, 2-methyl-1,3-propylene glycol, ethyl carbitol, 1,2-butylene glycol, and glycerin. From these, (D) an aqueous solvent can be appropriately selected for use in accordance with the kinds of (C) an oil component and (A) a hydrophilic nonionic surfactant. Furthermore, as (D) an aqueous solvent of the present invention, two or more of these can be used in combination.

Examples of preferable aqueous solvents include polypropylene glycol (1 to 20 mol) polyethylene glycol (5 to 30 mol) copolymer or its dimethyl ether, polyethylene glycol (5 to 30 mol) or its (C1 to C2)alkyl ethers, dipropylene glycol, isoprene glycol, and propylene glycol.

On the other hand, a water-soluble substance having four or more hydroxyl groups in the molecule normally becomes a solid at room temperature, and often cannot be used as (D) an aqueous solvent of the present invention.

The amount of (D) an aqueous solvent used in the present invention is not limited in particular; however, it is preferably 5 mass % or more with respect to the intermediate W/O emulsion. If the amount is less than 5 mass %, the preparation of a stable fine O/W emulsion tends to be difficult.

With respect to the combination of (C) an oil component and (D) an aqueous solvent used in the present invention, it is necessary that (D) an aqueous solvent is immiscible with (C) an oil component. Examples of such combinations are as follows:
When (C) the oil component is dimethylpolysiloxane, (D) the aqueous solvent is polypropylene glycol polyethylene glycol copolymer or its dimethyl ether, polyethylene glycol or its ethyl ether, 1,3-butylene glycol, dipropylene glycol, isoprene glycol, etc.
When (C) the oil component is cyclodimethicone (pentamer), (D) the aqueous solvent is polypropylene glycol polyethylene glycol copolymer or its dimethyl ether, polyethylene glycol or its ethyl ether, etc.
When (C) the oil component is methylphenylpolysiloxane, (D) the aqueous solvent is polypropylene glycol polyethylene glycol copolymer or its dimethyl ether, 1,3-butylene glycol, glycerin, etc.
When (C) the oil component is liquid paraffin, (D) the aqueous solvent is polypropylene glycol polyethylene glycol copolymer or its dimethyl ether, polyethylene glycol or its ethyl ether, 1,3-butylene glycol, dipropylene glycol, isoprene glycol, etc.

The amount of (E) water used in the present invention needs to be adjusted to 5 to 25 mass % with respect to the total amount of the intermediate W/O emulsion. If the amount is less than 5 mass %, the emulsion particle size of the final fine O/W emulsion is large. If the amount exceeds 25 mass %, the advantage of low-energy preparation is lost.

In addition, with the increasing value of the critical micelle concentration (c.m.c.) of (A) a hydrophilic nonionic surfactant in (D) an aqueous solvent, the amount of (E) water necessary for the production of the intermediate W/O emulsion tends to be smaller.

The fine O/W emulsion external preparation of the present invention is obtained by preparing a W/O emulsion, at about 70 to 80 °C, containing the above-described (A) to (E) as the essential components, and then adding (F) water or an aqueous formulation at 10 to 35 °C to the W/O emulsion.

(F) water or an aqueous formulation used in the present invention is not limited in particular so far as the main medium thereof is water or an aqueous solvent. In addition to water or an aqueous solvent, the components normally used in cosmetics, pharmaceuticals, etc. can be blended thereto in the quantity range that the stability is not affected.

The final amount of water in the fine O/W emulsion external preparation of the present invention is the sum of the amount of (E) water used for the formation of the W/O emulsion and the amount of water contained in (F) water or an aqueous formulation. The total amount of water used in the present invention is not limited in particular. Generally, the total amount of water is preferably 40 to 95 mass % with respect to the total amount of the fine O/W emulsion external preparation.

Furthermore, the temperature of (F) water or an aqueous formulation is preferably at 10 to 35 °C and more preferably at 15 to 35 °C. If the temperature is less than 10 °C, the emulsion particle size in the fine O/W emulsion external preparation is large, whereby the effect of the invention tends to be impaired. If the temperature exceeds 35 °C, the stability immediately after the production of the fine O/W emulsion external preparation tends to be poor.

Hereinafter, the concept of the production method of a fine O/W emulsion external preparation of the present invention will be explained.

### Concept:

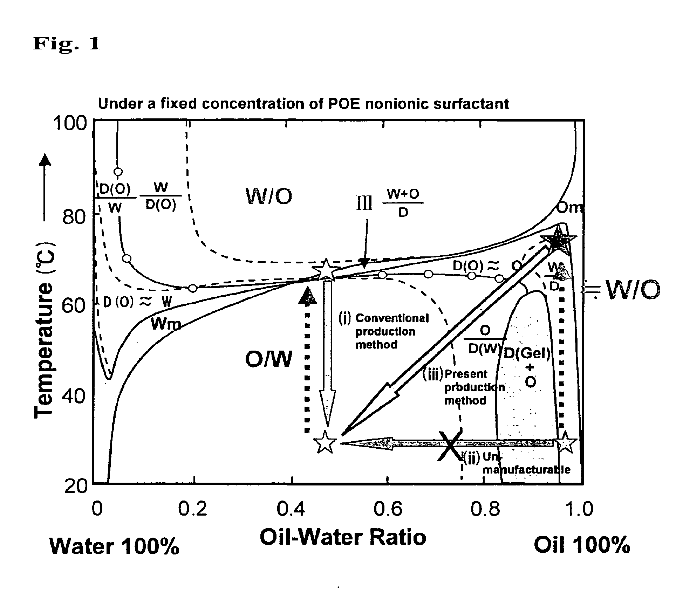

Fig. 1 shows one example of the phase diagram for a surfactant-oil-water system under a fixed concentration of the hydrophilic nonionic surfactant.

In the present invention, a W/O emulsion is obtained by mixing with stirring, at 70 to 80 °C, (A) a hydrophilic nonionic surfactant, (B) a linear higher alcohol having 16 or more carbon atoms, (C) an oil component, (D) an aqueous solvent that is freely soluble in water and provides a higher critical micelle concentration (c.m.c.) of the hydrophilic nonionic surfactant in the aqueous solvent than that in water, and (E) water in the amount of 5 to 25 mass % of the total amount of (A) to (E).

When (F) water or an aqueous formulation at 10 to 35 °C is gradually added to the W/O emulsion with stirring, as shown by the direction of the arrow in Fig. 1(iii), it enters into the O/W region without passing through the gelation region. Therefore, the phase inversion to a fine O/W emulsion, in which (C) the oil component is the inner phase, takes place without gelation, thereby obtaining a fine O/W emulsion.

The particle size of the obtained fine O/W emulsion external preparation is very small ranging 50 to 500 nm. Nevertheless, the external preparation is stable in a wide temperature range for a long period.

If a W/O emulsion is prepared, by mixing with stirring, at a lower temperature than 70 to 80 °C, the gelation takes place because the temperature is lower than the melting point of α-gel (temperature at the top of the gelation region in Fig. 1). Thus, even if the amount of (E) water is increased, or (F) water or an aqueous formulation is added to the W/O emulsion in this low temperature region, as shown in Fig. 1 (ii), a fine O/W emulsion external preparation of the present invention cannot be prepared.

In the W/O emulsion preparation process in the method of the present invention, a specific (D) aqueous solvent is blended in order to lower the melting point of the α-gel and decrease the viscosity. When such a specific (D) aqueous solvent is not blended, the gelation region is large and the melting point of α-gel is high, compared with when such a specific (D) aqueous solvent is blended. In such a case, when a hydrophobic W/O emulsion is prepared at a high temperature, 70 to 80 °C, and (F) water or an aqueous formulation at 10 to 30 °C is added to the W/O emulsion, the gelation takes place during the passage through the gelation region: thus, even if more (F) water or an aqueous formulation is added afterwards, a stable fine O/W emulsion of the present invention cannot be obtained anymore.

In the conventional O/W emulsion production method, as shown in Fig. 1(i), the following method has been used: water, a moisturizer, and a hydrophilic nonionic surfactant are dissolved in advance and heated to about 70 °C to prepare a water phase; the oil phase, which is obtained by uniformly mixing an oil component and a higher alcohol at about 70 °C, is emulsified by stirring with a homogenizer in the water phase; and then, the obtained emulsion is rapidly cooled to about 35 °C. However, the energy wastefulness is high in the conventional production method, and the consumption of water used for the cooling machine is large. In addition, it is difficult to prepare an emulsion having small particle sizes.

On the other hand, in the case of the fine O/W emulsion external preparation obtained by the production method of the present invention, the use of a cooling machine such as an Onlator and the heating of a large amount of (F) water or an aqueous formulation are not necessary during emulsification: thus, the O/W emulsion can be easily produced with low energy. In addition, since the O/W emulsion is substantially obtained by emulsification only with a nonionic surfactant whose irritation to the human body is relatively small, it has excellent safety. Furthermore, the O/W emulsion provides a light and fresh but also rich feeling in use, compared with the O/W emulsion produced from the identical components with the use of a cooling machine such as an Onlator: thus it has excellent usability.

As mentioned above, in the production method of a fine O/W emulsion external preparation of the present invention, an excellent fine O/W emulsion external preparation can be obtained only by blending water or an aqueous formulation to a pre-produced W/O emulsion; thus the conventional production process can be drastically simplified.

The fine O/W emulsion external preparation of the present invention can be used as products that can be applied to the body parts such as the skin and hair such as skin cosmetics, hair cleanser, skin cleanser, and hair styling preparations.

Furthermore, in the fine O/W emulsion external preparation of the present invention, in addition to the above-described essential components, the components normally used in cosmetics, pharmaceuticals, etc. can be blended in the quantity range that the stability is not affected. Examples of such components include the following.

Oil components such as avocado oil, macadamia nut oil, corn oil, olive oil, rapeseed oil, evening primrose oil, castor oil, sunflower oil, tea seed oil, rice bran oil, jojoba oil, cacao butter, coconut oil, squalene, beef tallow, Japan wax, beeswax, candelilla wax, carnauba wax, spermaceti wax, lanolin, liquid paraffin, polyoxyethylene (8 mol) oleyl alcohol ether, and glyceryl monooleate.

Higher alcohols such as capryl alcohol, lauryl alcohol, myristyl alcohol, cholesterol, and phytosterol.

Higher fatty acids such as capric acid, lauric acid, myristic acid, palmitic acid, stearic acid, behenic acid, lanolin fatty acid, linoleic acid, and linolenic acid.

Moisturizers such as polyethylene glycol and its alkyl ethers, glycerin, sorbitol, xylitol, maltitol, mucopolysaccharides, hyaluronic acid, chondroitin sulfate, and chitosan.

Thickeners such as methylcellulose, ethylcellulose, gum arabic, and polyvinyl alcohol.

Organic solvents such as ethanol and 1,3-butylene glycol.

Antioxidants such as butylhydroxytoluene, tocopherol, and phytic acid.

Antimicrobial preservative agents such as benzoic acid, salicylic acid, sorbic acid, paraoxybenzoic acid ester (ethylparaben, butylparaben, etc.), and hexachlorophene.

Amino acids such as glycine, alanine, valine, leucine, serine, threonine, phenylalanine, tyrosine, aspartic acid, asparagine, glutamine, taurine, arginine, and histidine; and their hydrochlorides.

Organic acids such as acyl sarcosinic acid (for example, sodium lauroyl sarcosinate), glutathione, citric acid, malic acid, tartaric acid, and lactic acid.

Vitamins including: vitamin A and its derivatives; vitamin Bs such as vitamin B6 hydrochloride, vitamin B6 tripalmitate, vitamin B6 dioctanoate, vitamin B2 and its derivatives, vitamin B12, and vitamin B15 and its derivatives; vitamin Cs such as ascorbic acid, ascorbyl phosphate (its salts), and ascorbyl dipalmitate; vitamin Es such as α-tocopherol, β-tocopherol, γ-tocopherol, vitamin E acetate, and vitamin E nicotinate; vitamin Ds; vitamin H; pantothenic acid; and pantethine. Various agents such as nicotinic acid amide, benzyl nicotinate, γ-oryzanol, allantoin, glycyrrhizic acid (its salts), glycyrrhetinic acid and its derivatives, hinokitiol, mucidin, bisabolol, eucalyptol, thymol, inositol, saponins (saikosaponin, ginseng saponin, luffa cylindrica saponin, sapindus mukorossi saponin, etc.), pantothenyl ethyl ether, ethinylestradiol, tranexamic acid, cepharanthine, and placenta extract.

Natural extracts, obtained by extraction with a solvent such as organic solvents, alcohols, polyhydric alcohols, water and aqueous alcohols, from materials such as sorrel, Sophora flavescens Aiton, cow lily, orange, sage, thyme, yarrow, mallow, cnidium root, Swertia japonica, Angelica acutiloba, spruce, birch, field horsetail, Luffa cylindrica, horse chestnut, saxifrage, arnica, lily, mugwort, peony, aloe, gardenia, sawara cypress, etc.

Cationic surfactants such as stearyltrimethylammonium chloride, benzalkonium chloride, and laurylamine oxide.

Sequestering agents such as disodium edetate, trisodium edetate, sodium citrate, sodium polyphosphate, sodium metaphosphate, and gluconic acid.

In addition, powder components, anionic surfactants, amphoteric surfactants, water-soluble polymers, thickeners, film-forming agents, UV absorbers, whitening agents, perfumes, scrubbing materials, etc. may be appropriately blended so far as the stability is not impaired.

### EXAMPLE 1

Hereinafter, the present invention will be explained in further detail by the examples of the fine O/W emulsion external preparations of the present invention. However, the present invention is not limited by these examples.

### Production Example 1-1

(1) A composition containing 5.5 mass % of polyoxyethylene (60 mol) glyceryl isostearate (manufactured by Nihon Emulsion Co., Ltd., EMALEX GWIS-160 (HLB 19)) as (A) a hydrophilic nonionic surfactant, 4.5 mass % of deodorized cetanol (manufactured by Kokyu Alcohol Kogyo Co., Ltd.) as (B) a linear higher alcohol having 16 or more carbon atoms, 10 mass % of liquid paraffin (manufactured by Nippon Oil Corporation, liquid paraffin E) as (C) an oil component, 5 mass % of dipropylene glycol as (D) an aqueous solvent, and 5 mass % of (E) water was heated to 70 °C and mixed with stirring, to obtain a W/O emulsion. [0053]
(2) Subsequently, (F) 70 mass % of water at 20 °C was gradually added to the W/O emulsion obtained in the above-described (1) while being stirred, thereby obtaining a faint blue-white fine O/W emulsion (Production Example 1-1).

When 20 mass % of water was added, the composition changed to a faint blue-white fine O/W emulsion.

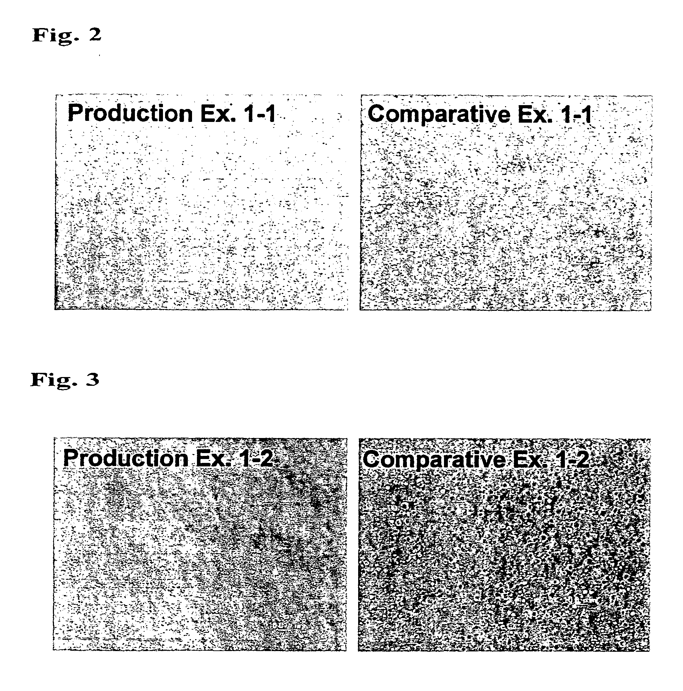

A 400 times magnification micrograph of the fine O/W emulsion of Production Example 1-1 is shown on the left side in Fig. 2. Based on the micrograph, the fine O/W emulsion obtained in the Production Example 1-1 was found to be a fine O/W emulsion having the particle size of about 300 nm.

### Comparative Example 1-1

(1) 5.5 mass % of polyoxyethylene (60 mol) glyceryl isostearate (manufactured by Nihon Emulsion Co., Ltd., EMALEX GWIS-160 (HLB19)) as (A) a hydrophilic nonionic surfactant, 5 mass % of dipropylene glycol as (D) an aqueous solvent, and 75 mass % of (E) water were heated to 70 °C and uniformly mixed.
(2) 4.5 mass % of deodorized cetanol (manufactured by Kokyu Alcohol Kogyo Co., Ltd.) as (B) a linear higher alcohol having 16 or more carbon atoms and 10 mass % of liquid paraffin (manufactured by Nippon Oil Corporation, liquid paraffin) as (C) an oil component were heated to 70 °C and uniformly mixed.
(3) The component (2) was added and emulsified into the component (1) at 70 °C while stirring with a homogenizer to prepare an O/W emulsion. The obtained O/W emulsion was passed through an Onlator to be cooled to 35 °C, thereby finally obtaining an O/W emulsion.

A 400 times magnification micrograph of the O/W emulsion of the Comparative Example 1-1 is shown on the right side in Fig. 2. Based on the micrograph, the O/W emulsion obtained in the Comparative Example 1-1 was found to be an O/W emulsion having the particle size of about 1 to 5 µm. When the micrographs in Fig. 2 are compared, it has been seen that a fine O/W emulsion with much smaller emulsion particle size can be produced in Production Example 1-1 though the formulation components are the same as those of Comparative Example 1-1.

### Production Example 1-2

Except for the use of 5 mass % of polyoxyethylene (14 mol) polyoxypropylene (7 mol) copolymer dimethyl ether as (D) an aqueous solvent, a similar operation to that of Production Example 1-1 was carried out, to obtain a faint blue-white fine O/W emulsion (Production Example 1-2).

When 25 mass % of (E) water was added, the composition changed to a stable fine O/W emulsion.

A 400 times magnification micrograph of the fine O/W emulsion of the Production

Example 1-2 is shown on the left side in Fig. 3. Based on the micrograph, the fine O/W emulsion obtained in the Production Example 1-2 was found to be a fine O/W emulsion having the particle size of about 100 nm.

### Comparative Example 1-2

Except for the use of 5 mass % of polyoxyethylene (14 mol) polyoxypropylene (7 mol) copolymer dimethyl ether as (D) an aqueous solvent, a similar operation to that of Comparative Example 1-1 was carried out.

A 400 times magnification micrograph of the O/W emulsion of the Comparative Example 1-2 is shown on the right side in Fig. 3. Based on the micrograph, the O/W emulsion obtained in the Comparative Example 1-2 was found to be an O/W emulsion having the particle size of about 1 to 10 µm. When the micrographs in Fig. 3 are compared, it has been seen that a fine O/W emulsion with much smaller emulsion particle size can be produced in Production Example 1-2 though the formulation components are the same as those of Comparative Example 1-2.

The evaluation results of the emulsion particles and stability for the O/W emulsions of the above-described Production Examples 1-1 and 1-2 and Comparative Examples 1-1 and 1-2 are summarized, with each blending composition, in the following Table 1. Here, the evaluation items are as follows, and the amounts in the following table are expressed by mass %.

### (Emulsion particle size of fine O/W emulsion)

For the O/W emulsion compositions formed in the Production Examples 1-1 and 1-2 and Comparative Examples 1-1 and 1-2, the particle sizes and micrographs were shown.

### (Stability of fine O/W emulsion)

The O/W emulsions formed in the Production Examples 1-1 and 1-2 and Comparative Examples 1-1 and 1-2 were stored at 50 °C for 1 month and then the stability was evaluated.
O: The O/W emulsion was not separated.
X: The O/W emulsion was separated.

**Table 1**

| | | Production Example 1-1 | Comparative Example 1-1 | Production Example 1-2 | Comparative Example 1-2 |
|---|---|---|---|---|---|
| (A) | Polyoxyethylene(60) glyceryl isostearate | 5.5 | 5.5 | 5.5 | 5.5 |
| (B) | Deodorized cetanol | 4.5 | 4.5 | 4.5 | 4.5 |
| (C) | Liquid paraffin | 10.0 | 10.0 | 10.0 | 10.0 |
| (D) | Dipropylene glycol | 5.0 | 5.0 | - | - |
| (D) | POE(14) POP(7) dimethyl ether | - | - | 5.0 | 5.0 |
| (E) | Ion-exchanged water | 5.0 | 5.0 | 5.0 | 5.0 |
| (F) | Ion-exchanged water | 70.0 | 70.0 | 70.0 | 70.0 |
| Eval | Emulsion Particles | 300nm | 1 to 5µm | 100nm | 1 to 10µm |
| | Stability | O | X | O | X |

As seen from the above Table 1 and Figs. 1 and 2, the production method of the present invention is very economical and easy because the O/W emulsion can be produced with low energy compared with the conventional process in which the Onlator is used for cooling. In addition, the emulsion had much finer particle size and stable.

Subsequently, in order to investigate the preferable temperature of (F) water or an aqueous formulation, which is added in the step of inversion to a fine O/W emulsion, the present inventors have investigated the stability of each emulsion by appropriately varying the temperature of (F) water, using the same formulation as that of Production Example 1-1 in the Table 1.

**Table 2**

| | | Production Examples | | | | | | | |
|---|---|---|---|---|---|---|---|---|---|
| | | 2-1 | 2-2 | 2-3 | 2-4 | 2-5 | 2-6 | 2-7 | 2-8 |
| Temperature of (F) Ion-exchanged water | | 5°C | 10°C | 15°C | 20°C | 25°C | 30°C | 35°C | 40°C |
| Eval | Emulsion Particles | 1 to 10µm | 300nm | 300nm | 300nm | 300nm | 300nm | 300nm | 1 to 10µm |
| | Stability | X | Δ | O | O | O | O | O | X |

As shown in the above Table 2, the temperature of (F) ion-exchanged water was appropriately varied in the production method of the present invention using the same blending formulation as the Production Example 1-1, to investigate the emulsion stability.

As a result, it was to be found that when the temperature of the added (F) ion-exchanged water was 10 to 35 °C, and in particular when the temperature was 15 to 35 °C (Production Examples 2-2 to 2-7), the emulsion particles were fine and excellent stability was provided.

On the other hand, when the temperature of (F) ion-exchanged water was 5 °C (Production Example 2-1), an emulsion with large particle sizes was formed; thus the desired fine O/W emulsion could not be obtained. When the temperature was 40 °C, the stability was also poor (Production Example 2-8).

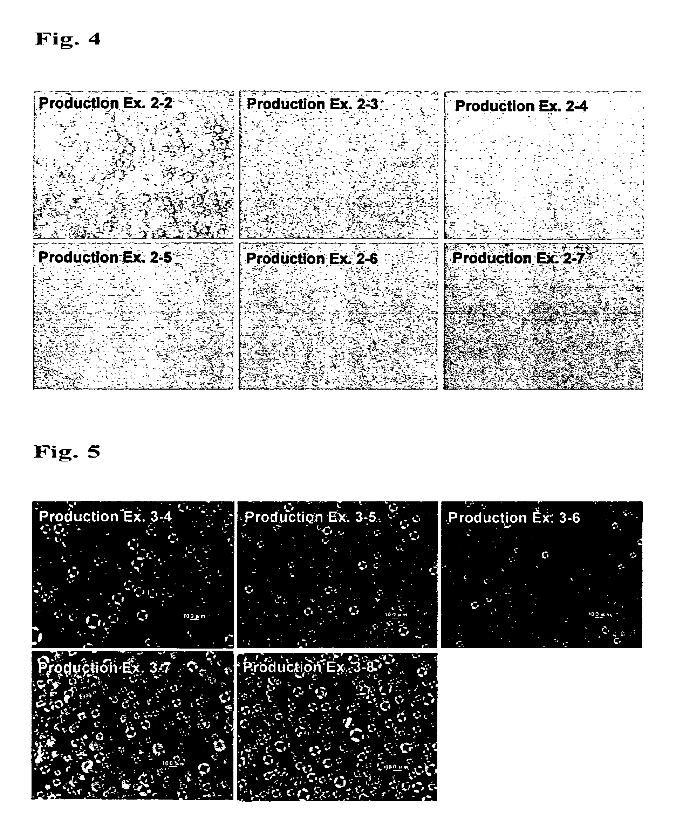

The micrographs for the O/W emulsions formed in the Production Examples 2-2 to 2-7 are shown in Fig. 4. As seen from these micrographs, the emulsion particle size was found to be extremely fine when the temperature of the added (F) ion-exchanged water was 10 to 35 °C.

Subsequently, the present inventors have investigated the mechanism of stability improvement of the O/W emulsion composition due to the combination of (A) a hydrophilic nonionic surfactant and (B) a linear higher alcohol having 16 or more carbon atoms. The production method was according to the Production Example 1-1.

**Table 3**

| | | Production Examples | | | | | | | | | | | |
|---|---|---|---|---|---|---|---|---|---|---|---|---|---|
| | | 3-1 | 3-2 | 3-3 | 3-4 | 3-5 | 3-6 | 3-7 | 3-8 | 3-9 | 3-10 | 3-11 | 3-12 |
| (A) | Polyoxyethylene (60) glyceryl isostearate | - | 2.0 | 3.0 | 3.5 | 4.0 | 4.5 | 5.0 | 5.5 | 6.0 | 7.0 | 8.0 | 10.0 |
| (B) | Deodorized cetanol | 10.0 | 8.0 | 7.0 | 6.5 | 6.0 | 5.5 | 5.0 | 4.5 | 4.0 | 3.0 | 2.0 | - |
| Ratio of (A) : (B) | | 0:10 | 2:8 | 3:7 | 3.5:6.5 | 4:6 | 4.5:5.5 | 5:5 | 5.5:4.5 | 6:4 | 7:3 | 8:2 | 10:0 |
| (C) | Liquid paraffin | 10.0 | 10.0 | 10.0 | 10.0 | 10.0 | 10.0 | 10.0 | 10.0 | 10.0 | 10.0 | 10.0 | 10.0 |
| (D) | Dipropylene glycol | 5.0 | 5.0 | 5.0 | 5.0 | 5.0 | 5.0 | 5.0 | 5.0 | 5.0 | 5.0 | 5.0 | 5.0 |
| (E) | Ion-exchanged water | 5.0 | 5.0 | 5.0 | 5.0 | 5.0 | 5.0 | 5.0 | 5.0 | 5.0 | 5.0 | 5.0 | 5.0 |
| (F) | Ion-exchanged water | 70.0 | 70.0 | 70.0 | 70.0 | 70.0 | 70.0 | 70.0 | 70.0 | 70.0 | 70.0 | 70.0 | 70.0 |
| Eval | Emulsion Particles | 5µm | 1µm | 300nm | 300nm | 200nm | 100nm | 200nm | 300nm | 300nm | 300nm | 10µm | Un-emulsifiable |
| | Stability | X | Δ | O | O | O | O | O | O | O | O | Δ | X |

As shown in the above Table 3, the total amount of (A) a hydrophilic nonionic surfactant and (B) a higher alcohol was set to be 10 mass % in Production Examples 3-1 to 3-12, but the blending ratio was changed, to investigate the stability of each emulsion.

As a result, when the blending ratio of (A) a hydrophilic nonionic surfactant and (B) a higher alcohol was 3:7 to 7:3 (Production Examples 3-3 to 3-10), the emulsion stability was excellent compared with when each component was used alone (Production Examples 3-1 and 3-12) or the blending ratio was 2:8 or 8:2 (Production Examples 3-2 and 3-11).

The micrographs of the O/W emulsion compositions formed in the Production Examples 3-4 to 3-8 were shown in Fig. 5. As seen from these micrographs, the emulsion particle size was found to be extremely fine when the blending ratio of (A) a hydrophilic nonionic surfactant and (B) a higher alcohol was 3:7 to 7:3.

Subsequently, the present inventors have investigated the preferable (D) aqueous solvent used for the preparation of a stable O/W emulsion. The production method was according to the Production Example 1-1.

**Table 4**

| | | Production Examples | | | | | | | |
|---|---|---|---|---|---|---|---|---|---|
| | | 4-1 | 4-2 | 4-3 | 4-4 | 4-5 | 4-6 | 4-7 | 4-8 |
| (A) | Polyoxyethylene (60) glyceryl isostearate | 5.5 | 5.5 | 5.5 | 5.5 | 5.5 | 5.5 | 5.5 | 5.5 |
| (B) | Deodorized cetanol | 4.5 | 4.5 | 4.5 | 4.5 | 4.5 | 4.5 | 4.5 | 4.5 |
| (C) | Liquid paraffin | 10.0 | 10.0 | 10.0 | 10.0 | 10.0 | 10.0 | 10.0 | 10.0 |
| (D)* | Polypropylene glycol (7) | 5.0 | - | - | - | - | - | - | - |
| | polyethylene glycol (14) | | | | | | | | |
| | copolymer dimethyl ether | | | | | | | | |
| | Polyethylene glycol | - | 5.0 | - | - | - | - | - | - |
| | Dipropylene glycol | - | - | 5.0 | - | - | - | - | - |
| | Isoprene glycol | - | - | - | 5.0 | - | - | - | - |
| | Propylene glycol | - | - | - | - | 5.0 | - | - | - |
| (D')* | Erythritol | - | - | - | - | - | 5.0 | - | - |
| | Maltitol | - | - | - | - | - | - | 5.0 | - |
| (E) | Ion-exchanged water | 5.0 | 5.0 | 5.0 | 5.0 | 5.0 | 5.0 | 5.0 | 5.0 |
| (F) | Ion-exchanged water | 70.0 | 70.0 | 70.0 | 70.0 | 70.0 | 70.0 | 70.0 | 70.0 |
| Eval | Emulsion Particles | 100nm | 300nm | 300nm | 200nm | 100nm | 10µm | 10µm | Un-emulsifiable (gelation) |
| | Stability | O | O | O | O | O | X | X | - |

| | | | | | | | | | |
|---|---|---|---|---|---|---|---|---|---|
| * cmc of (A) at 25°C in (D) > that in water > that in (D') | | | | | | | | | |

As shown in the above Table 4, when polypropylene glycol (7 mol) polyethylene glycol (14 mol) copolymer dimethyl ether, polyethylene glycol (molecular weight: 1000), dipropylene glycol, isoprene glycol, or propylene glycol, which is an aqueous solvent having three or less hydroxyl groups in the molecule and providing a higher c.m.c. of (A) a hydrophilic nonionic surfactant therein than the c.m.c. in water, was used (Production Examples 4-1 to 4-5), an O/W emulsion having fine emulsion particles and high stability could be obtained.

On the other hand, when erythritol (Production Example 4-6) or maltitol (Production Example 4-7), which has four or more hydroxyl groups in the molecule, was used, the composition became a hard gel during the step of the O/W emulsion preparation, and the desired fine O/W emulsion external preparation could not be obtained.

Furthermore, the present inventors have investigated the blending percentage of (E) water for the preparation of a fine O/W emulsion with excellent in stability. The production method was according to the Production Example 1-1.

**Table 5**

| | | Production Examples | | | | | | | |
|---|---|---|---|---|---|---|---|---|---|
| | | 5-1 | 5-2 | 5-3 | 5-4 | 5-5 | 5-6 | 5-7 | 5-8 |
| (A) | Polyoxyethylene (60) glyceryl isostearate | 5.5 | 5.5 | 5.5 | 5.5 | 5.5 | 5.5 | 5.5 | 5.5 |
| (B) | Deodorized cetanol | 4.5 | 4.5 | 4.5 | 4.5 | 4.5 | 4.5 | 4.5 | 4.5 |
| (C) | Liquid paraffin | 10.0 | 10.0 | 10.0 | 10.0 | 10.0 | 10.0 | 10.0 | 10.0 |
| (D) | Dipropylene glycol | 5.0 | 5.0 | 5.0 | 5.0 | 5.0 | 5.0 | 5.0 | 5.0 |
| (E) | Ion-exchanged water | 1.0 | 2.0 | 3.0 | 4.0 | 5.0 | 10.0 | 25.0 | 30.0 |
| (F) | Ion-exchanged water | 74.0 | 73.0 | 72.0 | 71.0 | 70.0 | 65.0 | 50.0 | 45.0 |
| Eval | Emulsion Particles | 10µm | 5µm | 5µm | 1µm | 300nm | 100nm | 200nm | 1µm |
| | Stability | X | X | X | Δ | O | O | O | X |

As shown in the above Table 5, the preferable blending percentage of (E) water for the preparation of the fine O/W emulsion of the present invention was investigated.

As a result, when the amount of (E) water was I to 4 mass %, the particle size was large and the stability had a tendency to be poorer (Production Examples 5-1 to 5-4). However, when the amount of (E) water was 5 to 25 mass %, the particle size of the fine O/W emulsion was very small and the stability was excellent (Production Examples 5-5 to 5-7). When the amount of (E) water was higher than 25 mass %, however, it has been clear to be found that the efficiency of emulsification turned out to be poor, and the advantage of low-energy preparation was lost because the volume to be heated became large (Production Example 5-8).

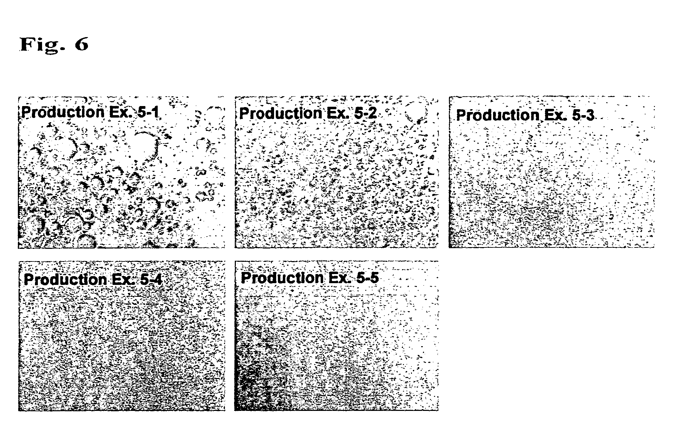

The micrographs of the O/W emulsions formed in the Production Examples 5-1 to 5-5 were shown in Fig. 6. As seen from these micrographs, the emulsion particle size was found to be extremely fine when the amount of (E) water was 5 mass % (Production Example 5-5).

### EXAMPLE 2

Hereinafter, the present invention will be further explained with reference to examples. However, the present invention is not limited by these examples. The quantities in the following formulation are expressed in mass %.

### Example 2-1

| Essence | Amount (mass %) |
|---|---|
| (1) Dimethylpolysiloxane | 1.2 |
| (Manufactured by Shin-Etsu Chemical Co., Ltd., Silicone KF96-A-6cs) | |
| (2) Behenyl alcohol | 0.7 |
| (3) Polyoxyethylene methylpolysiloxane copolymer | 0.76 |
| (Manufactured by Shin-Etsu Chemical Co., Ltd., Silicone KF6017) | |
| (4) Polyoxyethylene (20 mol) glyceryl isostearate | 0.5 |
| (Manufactured by Nihon Emulsion Co., Ltd., EMALEX GWIS-120) | |
| (5) 1,3-Butylene glycol | 1.0 |
| (6) Perfume | 0.1 |
| (7) Ion-exchanged water | 1.0 |
| (8) 1,3-Butylene glycol | 2.0 |
| (9) Glycerin | 4.0 |
| (10) Carboxyvinyl polymer | 0.03 |
| (11) Potassium hydroxide | 0.01 |
| (12) Tranexamic acid | 0.1 |
| (13) Ion-exchanged water | 88.6 |

Components (1) to (7) were heated to 75 °C and stirred to obtain a W/O emulsion. Aqueous components (8) to (13) were mixed at 25 °C and gradually added to the W/O emulsion while being stirred with a homogenizer, to obtain an essence. The emulsion particle size of the obtained essence was finer than that of the essence, of the identical formulation, obtained by separately heating the oil phase and the water phase at 75 °C, emulsifying them, and then cooling the emulsion with an Onlator. The obtained essence was stable and provided a light and fresh but also rich feeling in use.

### Example 2-2

| Cream | Amount (mass %) |
|---|---|
| (1) Liquid paraffin | 3.14 |
| (2) Diglyceryl diisostearate | 1.88 |
| (3) Polyoxyethylene (60 mol) glyceryl hydrogenated castor oil | 1.25 |
| (4) Behenyl alcohol | 3.0 |
| (5) Stearyl alcohol | 1.0 |
| (6) Perfume | 0.1 |
| (7) Dipropylene glycol | 3.0 |
| (8) Ion-exchanged water | 1.0 |
| (9) Glycerin | 5.0 |
| (10) Carboxyvinyl polymer | 1.0 |
| (11) Potassium hydroxide | 0.3 |
| (12) Chamomile extract | 0.1 |
| (13) Ion-exchanged water | 79.23 |

Components (1) to (8) were heated to 75 °C and stirred to obtain a W/O emulsion. Aqueous components (9) to (13) were mixed at 30 °C and gradually added to the W/O emulsion while being stirred with a homogenizer, to obtain a cream. The emulsion particle size of the obtained cream was finer than that of the cream, of the identical formulation, obtained by separately heating the oil phase and the water phase at 75 °C, emulsifying them, and then cooling the emulsion with an Onlator. The obtained cream was stable and provided a light and fresh but also rich feeling in use.

### Example 2-3

| Cleansing lotion | Amount (mass %) |
|---|---|
| (1) Decamethylcyclopentasiloxane | 12.1 |
| (Manufactured by Shin-Etsu Chemical Co., Ltd., KF-995) | |
| (2) Polyoxyethylene methylpolysiloxane copolymer | 1.88 |
| (Manufactured by Shin-Etsu Chemical Co., Ltd., Silicone KF6017) | |
| (3) Polyoxyethylene (20 mol) glyceryl isostearate | 1.25 |
| (Manufactured by Nihon Emulsion Co., Ltd., EMALEX GWIS-120) | |
| (4) Deodorized cetanol | 0.5 |
| (5) Perfume | 0.1 |
| (6) Polyethylene glycol 400 | 2.0 |
| (7) Ion-exchanged water | 1.0 |
| (8) Sodium lauroyl N-methyltaurate | 0.5 |
| (9) Ion-exchanged water | 80.67 |

Components (1) to (7) were heated to 70 °C and stirred to obtain a W/O emulsion. Aqueous components (8) to (9) were mixed at 25 °C and gradually added to the W/O emulsion while being stirred with a homogenizer, to obtain a cleansing lotion. The emulsion particle size of the obtained cleansing lotion was finer than that of the cleansing lotion, of the identical formulation, obtained by separately heating the oil phase and the water phase at 70 °C, emulsifying them, and then cooling the emulsion with an Onlator. The obtained cream was stable and provided good spreadability in use.

### Example 2-4

| Milky lotion | Amount (mass %) |
|---|---|
| (1) Pentaerythrityl tetraisostearate | 1.0 |
| (2) Behenyl alcohol | 0.4 |
| (3) Batyl alcohol | 0.2 |
| (4) Polyoxyethylene (20 mol) glyceryl isostearate | 0.91 |
| (5) Cholesteryl isostearate | 0.5 |
| (6) Polyoxyethylene (60 mol) glyceryl isostearate | 1.2 |
| (Manufactured by Nihon Emulsion Co., Ltd., EMALEX GWIS-160) | |
| (7) Polyoxyethylene (5 mol) glyceryl stearate | 0.8 |
| (Manufactured by Nihon Emulsion Co., Ltd., EMALEX GM-5) | |
| (8) Glyceryl tristearate | 2.5 |
| (9) Squalane | 4.5 |
| (10) Dimethylpolysiloxane | 1.0 |
| (Manufactured by Shin-Etsu Chemical Co., Ltd., Silicone KF96-A-6cs) | |
| (11) Perfume | 0.09 |
| (12) Dipropylene glycol | 7.0 |
| (13) Ion-exchanged water | 5.0 |
| (14) Erythritol | 1.3 |
| (15) Dynamite glycerin | 6.0 |
| (16) Phenoxyethanol | 0.3 |
| (17) Carboxyvinyl polymer | 0.12 |
| (18) Potassium hydroxide | 0.054 |
| (19) Sodium hexametaphosphate | 0.03 |
| (20) Ion-exchanged water | 67.096 |

Components (1) to (13) were heated to 75 °C and stirred to obtain a W/O emulsion. Aqueous components (14) to (20) were mixed at 20 °C and gradually added to the W/O emulsion while being stirred with a homogenizer, to obtain a milky lotion. The emulsion particle size of the obtained cream was finer than that of the milky lotion, of the identical formulation, obtained by separately heating the oil phase and the water phase at 75 °C, emulsifying them, and then cooling the emulsion with an Onlator. The obtained milky lotion was stable and provided good spreadability and rich feeling in use.

## Claims

1. A production method of a fine O/W emulsion external preparation having the emulsion particle size of 50 to 500 nm comprising the steps of:
mixing with stirring, at 70 to 80 °C,
(A) a hydrophilic nonionic surfactant,
(B) a linear higher alcohol having 16 or more carbon atoms,
(C) an oil component,
(D) an aqueous solvent which can be soluble in water and is immiscible with (C) the oil component, wherein the critical micelle concentration (c.m.c.) of the hydrophilic nonionic surfactant in the aqueous solvent is higher than that in water, and
(E) water in the amount of 5 to 25 mass % of the total amount of (A) to (E), to prepare a W/O emulsion; and
adding, while mixing with stirring, (F) water or an aqueous formulation at 10 to 35 °C into the W/O emulsion to invert the W/O emulsion to a fine O/W emulsion,
wherein
the mass ratio of (A) the hydrophilic nonionic surfactant and (B) the linear higher alcohol having 16 or more carbon atoms is 3:7 to 7:3, and the sum of (A) the hydrophilic nonionic surfactant and (B) the linear higher alcohol having 16 or more carbon atoms is 0.5 to 10 parts by mass with respect to the 10 parts by mass of (C) the oil component;
(C) the oil component is 1 to 40 mass % in the W/O emulsion; and
(D) the aqueous solvent is 5 mass % or higher in the W/O emulsion.

2. The production method of a fine O/W emulsion external preparation according to claim 1, wherein the HLB of (A) the hydrophilic nonionic surfactant is 8 or higher.

3. The production method of a fine O/W emulsion external preparation according to claim 1 or 2, wherein (D) the aqueous solvent has three or less hydroxyl groups in the molecule.

4. The production method of a fine O/W emulsion external preparation according to claim 3, wherein (D) the aqueous solvent is one or more selected from the group consisting of polypropylene glycol polyethylene glycol copolymer or its dimethyl ether, polyethylene glycol or its alkyl ethers, dipropylene glycol, isoprene glycol, and propylene glycol.

## Patentansprüche

1. Herstellungsverfahren für eine feine O/W-Emulsionszubereitung zur äußeren Anwendung mit einer Emulsionsteilchengröße von 50 bis 500 nm, umfassend die Stufen:
Mischen von
(A) einem hydrophilen nichtionischen grenzflächenaktiven Mittel,
(B) einem linearen höheren Alkohol mit 16 oder mehr Kohlenstoffatomen,
(C) einer Ölkomponente,
(D) einem wässrigen Lösemittel, das in Wasser löslich sein kann und mit (C), der Ölkomponente, nicht mischbar ist, wobei die kritische Mizellenkonzentration (c.m.c.) des hydrophilen nichtionischen grenzflächenaktiven Mittels in dem wässrigen Lösemittel höher als die in Wasser ist, und
(E) Wasser in einer Menge von 5 bis 25 Masse-% der Gesamtmenge von (A) bis (E)
unter Rühren bei 70 bis 80 °C zum Herstellen einer W/O-Emulsion und
Zugabe von (F) Wasser oder einer wässrigen Formulierung,
während des Mischens unter Rühren, bei 10 bis 35 °C in die W/O-Emulsion zum Invertieren der W/O-Emulsion in eine feine O/W-Emulsion,
wobei
das Masseverhältnis von (A), dem hydrophilen nichtionischen grenzflächenaktiven Mittel, und (B), dem linearen höheren Alkohol mit 16 oder mehr Kohlenstoffatomen, 3:7 bis 7:3 beträgt und die Summe von (A), dem hydrophilen nichtionischen grenzflächenaktiven Mittel, und (B), dem linearen höheren Alkohol mit 16 oder mehr Kohlenstoffatomen, 0,5 bis 10 Masseteile in Bezug auf die 10 Masseteile von (C), der Ölkomponente, beträgt;
(C), die Ölkomponente, 1 bis 40 Masse-% in der W/O-Emulsion beträgt und
(D), das wässrige Lösemittel, 5 Masse-% oder mehr in der W/O-Emulsion beträgt.

2. Herstellungsverfahren für eine feine O/W-Emulsionszubereitung zur äußeren Anwendung nach Anspruch 1, wobei der HLB-Wert von (A), dem hydrophilen nichtionischen grenzflächenaktiven Mittel, 8 oder mehr beträgt.

3. Herstellungsverfahren für eine feine O/W-Emulsionszubereitung zur äußeren Anwendung nach Anspruch 1 oder 2, wobei (D), das wässrige Lösemittel, drei oder weniger Hydroxylgruppen in dem Molekül aufweist.

4. Herstellungsverfahren für eine feine O/W-Emulsionszubereitung zur äußeren Anwendung nach Anspruch 3, wobei (D), das wässrige Lösemittel, eines oder mehrere ist, die aus der Gruppe von einem Polypropylenglykol-Polyethylenglykol-Copolymer oder dessen Dimethylether, Polyethylenglykol oder dessen Alkylethern, Dipropylenglykol, Isoprenglykol und Propylenglykol ausgewählt sind.

## Revendications

1. Procédé de production d'une préparation externe en émulsion huile-dans-eau (O/W) fine ayant une taille de particule en émulsion de 50 à 500 nm, comprenant les étapes consistant à :
mélanger sous agitation, à 70-80°C,
(A) un tensioactif non-ionique hydrophile,
(B) un alcool supérieur linéaire ayant 16 atomes de carbone ou plus,
(C) un composant huileux,
(D) un solvant aqueux qui peut être soluble dans l'eau et qui est non miscible avec le composant huileux (C), la concentration micellaire critique (c.m.c.) du tensioactif non-ionique hydrophile dans le solvant aqueux étant supérieure à celle dans l'eau, et
(E) de l'eau en une quantité de 5 à 25 % en masse de la quantité totale de (A) à (E), pour préparer une émulsion eau-dans-huile (W/O) ; et
ajouter, tout en mélangeant sous agitation,
(F) de l'eau ou une formulation aqueuse à 10-35°C dans l'émulsion W/O pour inverser l'émulsion W/O en une émulsion O/W fine,
dans lequel
le rapport en masse du tensioactif non-ionique hydrophile (A) à l'alcool supérieur linéaire ayant 16 atomes de carbone ou plus (B) est de 3/7 à 7/3, et la somme du tensioactif non-ionique hydrophile (A) et de l'alcool supérieur linéaire ayant 16 atomes de carbone ou plus (B) est de 0,5 à 10 parties en masse pour 10 parties en masse du composant huileux (C) ;
le composant huileux (C) représente de 1 à 40 % en masse de l'émulsion W/O ; et
le solvant aqueux (D)représente 5 % en masse ou plus de l'émulsion W/O.

2. Procédé de production d'une préparation externe en émulsion O/W fine selon la revendication 1, dans lequel l'indice HLB du tensioactif non-ionique hydrophile (A) est de 8 ou plus.

3. Procédé de production d'une préparation externe en émulsion O/W fine selon la revendication 1 ou 2, dans lequel le solvant aqueux (D) a trois ou moins de trois groupes hydroxyle par molécule.

4. Procédé de production d'une préparation externe en émulsion O/W fine selon la revendication 3, dans lequel le solvant aqueux (D) est un ou plusieurs solvants choisis dans le groupe constitué par un copolymère de polypropylèneglycol et de polyéthylèneglycol ou son diméthyléther, le polyéthylèneglycol ou ses alkyléthers, le dipropylèneglycol, l'isoprèneglycol et le propylèneglycol.
